# EUROPEAN PATENT APPLICATION

(11) **EP 4 716 376 A1**
(43) Date of publication of application: **25.03.2026**
(21) Application number: 24201624.4
(22) Date of filing: 20.09.2024
(51) Int. Cl.: H05B 47/16, A61N 5/06

(54) **INTENSE PULSED LIGHT (IPL) APPARATUS, ELECTRIC CIRCUIT, METHOD, AND COMPUTER PROGRAM PRODUCT**

(71) Applicant: Koninklijke Philips N.V., 5656 AG Eindhoven (NL)
(72) Inventor: VAN GOMPEL, Antonius Petrus Andreas Maria, Eindhoven (NL)
(74) Representative: Philips Intellectual Property & Standards

(57) **Abstract**

There is provided an intense pulsed light, IPL, apparatus. The IPL apparatus comprises an IPL light source, a power source, a switch, and a controller. The IPL light source is adapted to generate a pulse of light in response to receiving an electric current. The power source is adapted to provide the electric current to the IPL light source. The switch is electrically connected to the power source and the IPL light source. The controller is configured to provide a pulse modulation signal to the switch to open and close the switch. The switch is adapted to provide pulsed delivery of the electric current to the IPL light source in response to the pulse modulation signal. The controller is configured to control a frequency content of the pulse modulation signal to spread an electromagnetic emission caused by operation of the switch over multiple frequencies.

## Description

### FIELD OF THE INVENTION

The invention relates to an intense pulsed light (IPL) apparatus. The invention further relates to an electric circuit for use in an IPL apparatus. The invention further relates to a method for operating a switch of an IPL apparatus.

### BACKGROUND OF THE INVENTION

Photo-epilation is well known for hair removal and hair growth reduction. Home-use consumer devices are commercially available, such as the Lumea (TM) of Philips (TM). Home-use devices typically use Intense Pulsed Light technology (IPL) from e.g. a Xenon flash lamp at a relatively low fluence (up to 6.5 J/cm²), as compared to professional devices for permanent photo-epilation, that use fluences in excess of 10 J/cm².

IPL technology uses a high-powered, hand-held, flash lamp to deliver an intense, visible, broad-spectrum pulse of light, generally in the visible spectral range of 400 to 1200 nm. Cutoff filters are for example used to selectively filter out shorter wavelengths, especially potentially damaging ultra violet light. The resulting light has a spectral range that targets specific structures and chromophores, in particular the melanin pigment in hair.

The light absorbed by melanin in the hair and hair matrix generates heat that puts the hair follicles in a sleep state. When the treatment is repeated in intervals of 2 to 4 weeks, a long-lasting hair reduction result is obtained.

US patent application US 2005/0107850 A1 discloses a system for providing non-coherent pulsed light. The system includes a lamp to produce non-coherent light energy in a pulsed mode, a current supply to provide energy to the system, and a switching module to control the spectral distribution and/or light intensity in the non-coherent pulsed light energy during a pulse of non-coherent light. The switching module is adapted to modulate the power supply provided to the lamp to effect changes in spectral distribution and/or light intensity emitted from the lamp.

### SUMMARY OF THE INVENTION

The switching that generates the modulation of the power supply is in the range of several kHz up to several MHz. As a result, the switching produces electromagnetic noise that possibly causes disturbances in other electronic equipment. The power level of the electromagnetic noise needs to be below a level as set by regulations to limit the effect of the electromagnetic noise on other electronic equipment. The level as set by regulation is further referred to as a safety level. It is well-known to use filters to reduce electromagnetic noise. However, implementing such filters increases the costs and the volume of the apparatus.

It is an objective of the invention to reduce the electromagnetic noise generated by the IPL apparatus in a cost-effective way.

According to a first aspect of the invention, there is provided an intense pulsed light, IPL, apparatus. The IPL apparatus comprises an IPL light source, a power source, a switch, and a controller. The IPL light source is adapted to generate a pulse of light in response to receiving an electric current. The power source is adapted to provide the electric current to the IPL light source. The switch is electrically connected to the power source and the IPL light source. The controller is configured to provide a pulse modulation signal to the switch to open and close the switch. The switch is adapted to provide pulsed delivery of the electric current to the IPL light source in response to the pulse modulation signal. The controller is configured to control a frequency content of the pulse modulation signal to spread an electromagnetic emission caused by operation of the switch over multiple frequencies.

The operation of the switch causes the electromagnetic emission. The power level of the electromagnetic emission is a function of the frequency of the electromagnetic emission. The power level for each frequency depends on the frequency content of the pulse modulation signal. Without any control of the frequency content of the pulse modulation signal, the electromagnetic emission would have a high power level in a narrow frequency band, for example at the frequency at which the pulses are started. Such a high power level would exceed the safety level. By controlling the frequency content of the pulse modulation signal, the power of the electromagnetic emission is spread over the multiple frequencies. This results in a lower maximum power level of the electromagnetic emission. As a result, the electromagnetic emission is reduced to an acceptable level without the need for any additional filter. The electromagnetic noise generated by the IPL apparatus is reduced in a cost-effective way.

The IPL light source is adapted to provide a pulse of light. For example, the IPL light source is adapted to generate light at a high intensity for a short duration, such as for less than 1 second or less than 0.5 seconds. The intensity of the light pulse is high enough to perform an operation on the skin. Such operation is, for example, hair removal or photo-rejuvenation or vein treatment or acne treatment. For example, the IPL light source comprises one or more optical filters. For example, the IPL light source comprises an optical filter to prevent light with a potentially damaging wavelength, such as UV light, from being transmitted to the skin. For example, the IPL light source comprises a gas discharge flash lamp, or a laser, or multiple lasers. For example, the gas discharge flash lamp is a Xenon flash lamp. For example, the IPL light source is adapted to provide IPL light with wavelengths in the range of 530-1200 nanometers.

The power source comprises, for example, a capacitor. A capacitor is suitable to store sufficient electrical energy for the pulse of light, and is able to provide the electrical energy sufficiently fast to the IPL light source. For example, the capacitor has a capacity of 100 J of energy at 400 V. For example, the capacitor has a capacitance in the order of mF, such as 0.5 mF to 10 mF, for example 1.4 mF. For example, the power source comprises a battery to provide electrical power to the capacitor. For example, the battery is adapted to provide electrical power to the capacitor when the capacitor is not being discharged to the IPL light source. When the capacitor discharges, the electrical current from the capacitor is used by the IPL light source to generate the pulse of light. The time between successive pulses of light is sufficient for the battery to charge the capacitor. For example, the power source comprises a transformer to transform electric power from a low voltage of the battery to a high voltage of the capacitor. For example, the power source comprises a starter circuit adapted to start the IPL light source, whereas the capacitor provides the majority of the energy for the pulse of light after the starter circuit has started the IPL light source. For example, the starter circuit comprises a boost capacitor and/or a plasma ignition unit.

The switch comprises, for example, a field-effect transistor (FET), such as a metal-oxide-semiconductor field-effect transistor (MOSFET), or a metal-insulator-semiconductor field-effect transistor (MISFET), or an insulated-gate field-effect transistor (IGFET). The switch comprises, for example, a transistor, such as an insulated-gate bipolar transistor (IGBT) or a bipolar junction transistor (BJT). The switch comprises, for example, a relay.

The switch is referred to as being "closed" when the switch is in a state that allows an electric current to flow through the switch. The switch is referred to as being "open" when the switch is in a state that blocks any electric current from flowing through the switch. For example, the switch is arranged in series with the power source and the IPL light source. For example, the switch is arranged between the power source and the IPL light source. For example, the switch is arranged between the IPL light source and the ground. In case the switch is arranged between the IPL light source and the ground, the switch prevents the electric current to flow between the power source and the IPL light source because the electric current is not able to flow to the ground.

The controller comprises, for example, a microcontroller, a microprocessor, an application specific integrated circuits (ASIC), a complex programmable logic device (CPLD), a programmable array logic (PAL), a programmable logic array (PLA), and/or a field-programmable gate array (FPGA). For example, the pulse modulation signal directly controls the switch. In another example, the pulse modulation signal causes the switch to generate an actuation signal. The actuation signal causes the control of the switch. The controller comprises, for example, an internal clock to provide a clock signal. The controller generates the pulse modulation signal based on the clock signal.

For example, the pulse modulation signal is based on only two values: a high value and a low value. For example, the high value is 1, and the low value is 0 or -1. For example, in response to the high value, the switch is closed. In response to the low value, the switch is open.

The switch is adapted to provide the pulsed delivery of the electric current to the IPL light source by interrupting the electric current by repeatedly opening and closing. By providing the pulsed delivery, the pulse of light has a lower initial intensity peak compared to providing the electric current in a single delivery. The lower initial intensity peak improves the comfort for the user, as the lower initial intensity peak results in less heat and less irritation to the skin of the user.

In case the invention is not applied, the pulses are, for example, generated at a frequency of 100 kHz. As a result, every 10 µs a new pulse is generated. The causes an electromagnetic emission with a high power at 100 kHz. By controlling the frequency content of the pulse modulation signal according to the invention, the timing of the pulses in the pulse modulation signal are varied. For example, the controller generates one pulse or a group of pulses with a 10 µs time period, followed by one pulse or a group of pulses with a 9 µs time period. The pulses with the 10 µs time period create an electromagnetic emission at 100 kHz, whereas the pulses with the 9 µs time period create an electromagnetic emission at 111 kHz. This way, the electromagnetic emission is spread over the frequencies 100 kHz and 111 kHz. As a result, the power level at each frequency is below the safety level. For example, the time periods of the pulses change during the pulse of light at least 4 times or at least 5 times or at least 10 times or at least 100 times or at least 1000 times to spread the electromagnetic emission caused by operation of the switch over multiple frequencies.

In the example above, two frequencies are used, i.e., 100 kHz and 111 kHz. In another example, two different frequencies are used and/or more than two frequencies are used, such as 3 or 5 or 10 or 50 or 100 frequencies.

In an embodiment, wherein the pulse modulation signal comprises a spread spectrum modulation.

According to this embodiment, spread spectrum modulation is used to spread the frequencies at which the switch is operated over a larger frequency range. As a result, the electromagnetic noise is spread over the larger frequency range, causing the maximum power level of the electromagnetic noise to be reduced. For example, the spread spectrum modulation comprises direct sequence spread spectrum modulation, or frequency hopping, or time-hopping spread spectrum modulation, or chirp spread spectrum modulation, or any combination of these techniques.

In an embodiment, the controller is configured to control the frequency content by controlling start times of pulses of the pulse modulation signal.

The start times of the pulses of the pulse modulation signal have a significant influence on the frequency content of the pulse modulation signal. By controlling the start times, the start time is varied to spread the frequency content of the pulse modulation signal over a larger range of frequencies. As a result, the electromagnetic emission caused by the operation of the switch is spread over multiple frequencies. For example, the duty cycle of each pulse is unaffected by the varying the start times. For example, in a series of pulses, each of the pulses has a different start time. The duty cycle for all the series of pulses remains the same. To achieve this, the controller adapts the pulse width in dependency of the time periods as defined between two start times. In case the time period is reduced due to the varying start time, the pulse width is reduced to obtain the same duty cycle. In case the time period is extended due to the varying start time, the pulse width is extended to obtain the same duty cycle.

In an embodiment, the pulse modulation signal has a first pulse during a first time period, and a second pulse during a second period adjacent to the first time period. The switch is adapted to close and open in response to the first pulse. The switch is adapted to close and open in response to the second pulse. The controller is configured to set a duration of the first time period different from a duration of the second time period.

According to this embodiment, the controller is configured to set the duration of the first time period and the second time period differently from each other. As a result of the different durations, the frequency content of the first pulse and the second pulse is different from each other. This causes the electromagnetic noise to be spread over the multiple frequencies, reducing the maximum power level of the electromagnetic noise.

In an embodiment, the pulse modulation signal has a pulse width for each pulse in the pulse modulation signal. The controller is configured to control the frequency content by controlling the pulse widths.

According to this embodiment, the controller is configured to set the duration of the pulse widths differently from each other. As a result of the different durations, the frequency content of the pulses is spread over multiple frequencies. This causes the electromagnetic noise to be spread over the multiple frequencies, reducing the maximum power level of the electromagnetic noise.

In an embodiment, the pulse modulation signal has a pulse width for each pulse in the pulse modulation signal. The controller is configured to control the pulse width to control an intensity of the pulse of light.

The pulse width is a measure of the time between the leading edge and the trailing edge of a pulse in the pulse modulation signal. The pulse width defines the time the switch is closed, allowing current to flow to the IPL light source. Increasing the pulse width increases the time current flows to the IPL light source, and thus the intensity of the pulse of light is increased. Decreasing the pulse width decreases the time current flows to the IPL light source, and thus the intensity of the pulse of light is decreased. For example, by controlling the pulse width, the duty cycle of the pulse is controlled. The duty cycle is the ratio between the pulse width and the time period of the pulse. The duty cycle is in a range between 0-100%. With a duty cycle of 0, the pulse width is 0. With a duty cycle of 100%, the pulse width extends over the entire time period of the pulse. Increasing the duty cycle increases the time current flows to the IPL light source, and thus the intensity of the pulse of light is increased. Decreasing the duty cycle decreases the time current flows to the IPL light source, and thus the intensity of the pulse of light is decreased.

In an embodiment, the multiple frequencies form a range of frequencies between a first frequency and a second frequency. The controller is configured to control the frequency content by gradually changing the pulse modulation signal from the first frequency to the second frequency.

According to this embodiment, the controller controls the pulse modulation signal to start generating pulses with a frequency content of the first frequency. Then, the controller gradually changes the frequency content from the first frequency to the second frequency. As a result, the pulses gradually change to have a frequency content towards the second frequency. For example, the controller gradually changes the pulse modulation signal from the first frequency to the second frequency in a linear way, or in a triangular way, or in a sine-shaped way. In the linear way, the frequency content changes from the first frequency to the second frequency directly proportional with the duration of the pulse. So the frequency content is at the first frequency at the start of the pulse, is halfway the first frequency and the second frequency at the middle of the pulse, and is at the second frequency at the end of the pulse. The linear way distributes the frequency content evenly over all frequencies between the first frequency and the second frequency. In the sine-shaped way, the frequency content changes according to half a sine wave. In the sine-shaped way, the frequency content changes slowly near the start and the end of the pulse, but changes rapidly at the middle of the pulse. The sine-shaped way distributes the frequency content over all frequencies between the first frequency and the second frequency, but for the major part over frequencies near the first frequency and near the second frequency. The triangular way is, for example, the same as the linear way or the sine-shaped way, except that the controller gradually changes the frequency content from the first frequency to the second frequency and then gradually back from the second frequency to the first frequency. For example, the controller is configured to sweep from the first frequency to the second frequency. For example, the first frequency is higher than the second frequency. For example, the first frequency is lower than the second frequency. For example, the second frequency is 1%, or 2%, 5%, or 10% or 15% or 20% different than the first frequency.

In an embodiment, the multiple frequencies form a range of frequencies between a first frequency and a second frequency. The controller is configured to: control the frequency content by selecting frequencies from the range of frequencies in a pseudo-random way, and control start times of pulses of the pulse modulation signal based on the selected frequencies.

According to this embodiment, the controller selects in a pseudo-random way a frequency from the range of frequencies, and controls the start time based on that selected frequency. As a result, the start times are varied in a pseudo-random way. This causes the frequency content of the pulse modulation signal to be distributed over the range of frequencies between a first frequency and a second frequency. For example, the controller comprises a pseudo-random generated adapted to implement an algorithm for generating a sequence of numbers whose properties approximate properties of a sequence of random numbers. The controller controls the start times based on the sequence of numbers.

In an embodiment, the IPL apparatus comprises a clock connected to the controller. The clock provides a modulated clock signal. The controller is configured to provide the pulse modulation signal based on the modulated clock signal.

According to this embodiment, the modulated clock signal has a frequency content distributed over a range of frequencies. For example, the clock uses any of the ways discussed above to have the frequency content distributed over the range of frequencies. The modulated clock signal represents a series of time periods. Because the modulated clock signal is modulated, the time periods have durations different from each other. The controller uses the modulated clock signal to generate the pulse modulation signal. Because of the modulated clock signal, the implementation of the controller may be simplified, as the controller does not need to generate any modulation, but instead uses the modulated clock signal as provided by the clock.

In an embodiment, the frequency content is in the range of 10 kHz and higher, for example, 10 kHz to 500 kHz, for example 50 kHz to 500 kHz.

According to this embodiment, the frequency content is in the range of 10 kHz and higher. By operating the switch with at least 10 kHz, an intensity peak of the pulse of light is substantially reduced compared to operating the switch without modulation. For example, the switch is operated at at least 50 kHz, such as up to 500 kHz. There is a wide selection of commercially available, cost-effective switches that are able to switch up to 500 kHz.

In an embodiment, a duration of the pulse of light is in the range of 2 ms to 50 ms.

When using the IPL apparatus for hair removal, the duration of the pulse of light affects the effectiveness of removing hair. Depending on the properties of the hair, such as the color or thickness, some hairs are removed more effectively using short pulses of light, whereas other hairs are removed more effectively using long pulses of light.

In a second aspect of the invention, there is provided an electric circuit for use in an intense pulsed light, IPL, apparatus. The IPL apparatus has an IPL light source adapted to generate a pulse of light in response to receiving an electric current. The electric circuit comprises a power source, a switch, and a controller. The power source is adapted to provide the electric current to the IPL light. The switch is electrically connected to the power source and the IPL light source. The controller is configured to provide a pulse modulation signal to the switch to open and close the switch. The switch is adapted to provide pulsed delivery of the electric current to the IPL light source in response to the pulse modulation signal. The controller is configured to control a frequency content of the pulse modulation signal to spread an electromagnetic emission caused by operation of the switch over multiple frequencies.

In a third aspect of the invention, there is provided a method for controlling a switch of an intense pulsed light, IPL apparatus. The switch is electrically connected to a power source and an IPL light source of the IPL apparatus. The IPL light source is adapted to generate a pulse of light in response to receiving an electric current from the power source. The power source is adapted to provide the electric current to the IPL light source. The method comprises: providing a pulse modulation signal to the switch to open and close the switch; operating the switch based on the pulse modulation signal to provide pulsed delivery of the electric current to the IPL light source; and controlling a frequency content of the pulse modulation signal to spread an electromagnetic emission caused by operation of the switch over multiple frequencies.

In an embodiment, the pulse modulation signal comprises a spread spectrum modulation.

In an embodiment, wherein controlling the frequency content comprises controlling start times of pulses of the pulse modulation signal.

In a fourth aspect, there is provided a computer program product, comprising instructions which, when executed by a controller of an IPL apparatus, cause the controller to carry out the method of the third aspect of the invention.

In a fifth aspect, there is provided a computer-readable storage medium, comprising instructions which, when executed by a controller of an IPL apparatus, cause the controller to carry out the method of the third aspect of the invention.

### BRIEF DESCRIPTION OF THE DRAWINGS

Exemplary embodiments will now be described, by way of example only, with reference to the following Figures, in which:
FIG. 1 depicts an IPL apparatus according to a first embodiment of the invention;
FIG. 2 depicts an electric circuit for use in the IPL apparatus according to the first embodiment;
FIG. 3 depicts a light intensity during a pulse of light when switching without modulation;
FIG. 4 depicts a light intensity during a pulse of light when switching using modulation;
FIG. 5 depicts electromagnetic noise;
FIG. 6 depicts an example of the pulse modulation signal;
FIG. 7 depicts another example of the pulse modulation signal;
FIG. 8 depicts a controller according to a second embodiment;
FIG. 9 depicts a method for controlling a switch of the IPL apparatus according to a third embodiment.

### DETAILED DESCRIPTION OF EMBODIMENTS

It should be understood that the detailed description and specific examples, while indicating exemplary embodiments of the apparatus and methods, are intended for purposes of illustration only and are not intended to limit the scope of the invention. These and other features, aspects, and advantages of the apparatus and method of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

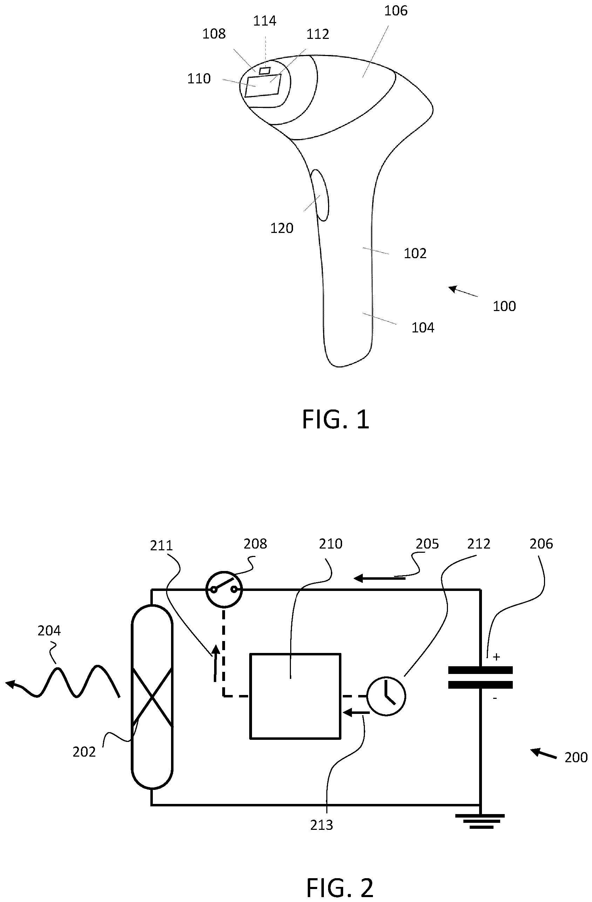

FIG. 1 depicts an Intense Pulsed Light, IPL, apparatus 100 according to a first embodiment of the invention. The IPL apparatus 100 is adapted for use on the skin of a subject, e.g., a person or an animal. The IPL apparatus 100 comprises a housing 102 that has a handle portion 104 and a head portion 106. The handle portion 104 is shaped to enable the user to hold the IPL apparatus 100. The head portion 106 has a head end 108 that is to be placed into contact with the subject in order for the IPL apparatus 100 to perform a treatment operation on the skin of the subject.

The IPL apparatus 100 comprises an IPL light source 202 adapted to provide IPL light to the skin. The IPL light source 202 is shown in FIG. 2. The IPL light source 202 is arranged inside the head portion 106. The IPL apparatus 100 performs the treatment operation by providing pulses of the IPL light to the skin via the aperture 110. The aperture 110 is arranged in or on the housing 102 so that the aperture 110 can be placed adjacent to or on (i.e. in contact with) the skin of the subject. An IPL window 112 is arranged in the aperture 110. The intensity of the light pulses from the IPL light source 202 is high enough to perform the treatment operation on the skin or body part adjacent to the aperture 110. Optionally, the IPL apparatus 100 comprises a sensor system for measuring a characteristic of the skin, such as skin tone, via window 114.

The IPL apparatus 100 comprises a user control 120 adapted to be operated by the user to activate the IPL apparatus 100. When the user activates the IPL apparatus 100 via the user control 120, the IPL apparatus 100 is able to perform the treatment operation. The user control 120 may be in the form of a button, a touch pad, etc.

FIG. 2 depicts an electric circuit 200 for use in the IPL apparatus 100 according to the first embodiment. The IPL apparatus 100 has the IPL light source 202 adapted to generate a pulse of light 204 in response to receiving an electric current 205. The electric circuit 200 comprises a power source 206, a switch 208 and a controller 210. The power source 206 is adapted to provide the electric current 205 to the IPL light. The switch 208 is electrically connected to the power source 206 and the IPL light source 202. In the Figure, the switch 208 is arranged between the power source 206 and the IPL light source 202. Alternatively, the switch 208 is arranged between the IPL light source 202 and the ground. The controller 210 is configured to provide a pulse modulation signal 211 to the switch 208 to open and close the switch 208. The switch 208 is adapted to provide pulsed delivery of the electric current 205 to the IPL light source 202 in response to the pulse modulation signal 211. The controller 210 is configured to control a frequency content of the pulse modulation signal 211 to spread an electromagnetic emission caused by operation of the switch 208 over multiple frequencies.

In an example, the IPL apparatus 100 comprises the IPL light source 202, the power source 206, the switch 208 and the controller 210. The IPL light source 202 is adapted to generate a pulse of light 204 in response to receiving an electric current 205. The power source 206 is adapted to provide the electric current 205 to the IPL light source 202. The switch 208 is electrically connected to the power source 206 and the IPL light source 202. The controller 210 is configured to provide a pulse modulation signal 211 to the switch 208 to open and close the switch 208. The switch 208 is adapted to provide pulsed delivery of the electric current 205 to the IPL light source 202 in response to the pulse modulation signal 211. The controller 210 is configured to control the frequency content of the pulse modulation signal 211 to spread an electromagnetic emission caused by operation of the switch 208 over multiple frequencies.

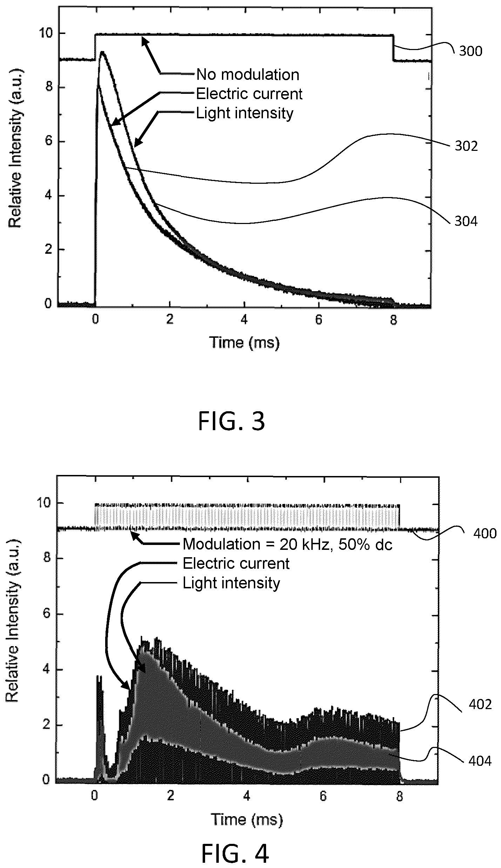

FIG. 3 depicts the light intensity during a pulse of light 204 when switching without modulation. Line 300 indicates the status of the switch 208. The switch 208 is open before t=0 ms. At t=0 ms, the switch 208 is closed, allowing the electric current 205 to flow through the IPL light source 202. At t=8 ms, the switch 208 is opened, interrupting the electric circuit 200. In response to closing the switch 208, the electric current 205 flows as indicated with line 302. The power source 206 comprises a capacitor. At t=0 ms, the capacitor is fully charged, which causes a large initial peak of the electric current 205. After the peak, the amperage of the electric current 205 reduces gradually to about 0 at t=8 ms. In response to the electric current 205, the IPL light source 202 generates the pulse of light 204 with the intensity as indicated with line 304. Due to the peak of the electric current 205, the pulse of light 204 has an intensity peak at shortly after t=0 ms. After the intensity peak, the intensity exponentially decreases to about a low value at t=8 ms. The intensity peak causes discomfort or even pain to the user of the IPL apparatus 100.

The duration of the pulse of light 204 is 8 ms, which is an example of the duration of the pulse of light 204 in the range of 2 ms to 50 ms.

FIG. 4 depicts the light intensity during a pulse of light 204 when switching using modulation. Line 400 indicates the modulation with which the switch 208 is opened and closed. The modulation is at 20 kHz with a duty cycle of 50% dc. Because of the modulation, the peak of the electric current 205 is substantially reduced, as shown with line 402. In response to the electric current 205, the IPL light source 202 generates the pulse of light 204 with the intensity as indicated with line 404. Because of the reduction of the peak in the electric current 205, the intensity peak of the pulse of light 204 is also reduced significantly compared to the peak in FIG. 3.

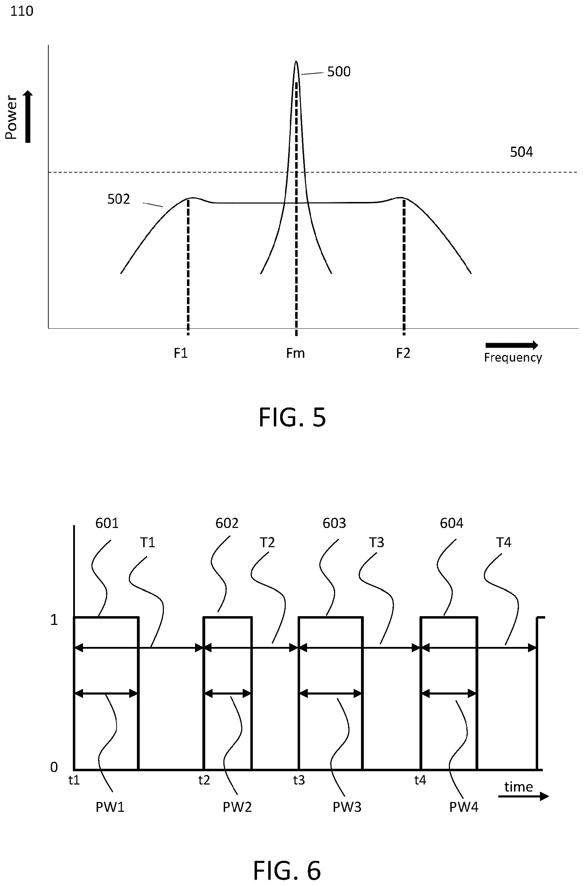

However, the switching with the modulation at 20 kHz causes electromagnetic noise with a large power level at or near 20 kHz, see FIG. 5. The modulation frequency of 20 kHz is indicated with frequency Fm. The power level of the 20 kHz modulation is indicated with line 500 and exceeds a safety level 504.

By controlling the frequency content of the pulse modulation signal 211 to spread the electromagnetic emission caused by operation of the switch 208 over multiple frequencies, the power level of the electromagnetic noise is reduced as depicted with line 502. The power of the electromagnetic noise is distributed over a range of frequencies between a first frequency F1 and a second frequency F2.

For example, the frequency content is in the range of 10 kHz and higher, for example, 10 kHz to 500 kHz, for example 50 kHz to 500 kHz. For example, the first frequency F1 is 10 kHz or higher. For example, the second frequency F2 is 500 kHz or lower.

For example, the pulse modulation signal 211 comprises a spread spectrum modulation.

FIG. 6 depicts an example of the pulse modulation signal 211. FIG. 6 depicts four pulses 601-604 of the pulse modulation signal 211. Pulse 601 has time period T1 and pulse width PW1, pulse 602 has time period T2 and pulse width PW2, pulse 603 has time period T3 and pulse width PW3, and pulse 604 has time period T4 and pulse width PW4. The pulses 601-604 have an amplitude of 1. In response to the pulses 601-604, the switch 208 closes to allow the electric current 205 to flow via the switch 208. When the pulse modulation signal 211 is 0 in between the pulses 601-604, the switch 208 is open to block the electric current 205 to flow via the switch 208.

The controller 210 is configured to control the frequency content by controlling start times of the pulses 601-604. The controller 210 starts pulse 601 at time t1. After the time period T1, the controller 210 starts pulse 602 at time t2. After time period T2, the controller 210 starts pulse 603 at time t3. Time period T1 is different from time period T2, in this case shorter. The controller 210 makes the time period T2 shorter by starting the pulse 603 at time t3. After the time period T3, the controller 210 starts the pulse 604 at time t4. Time period T3 is different from time period T2, in this case longer. The controller 210 makes the time period T3 longer by starting the pulse 604 at time t4.

As shown in FIG. 6, the pulse modulation signal 211 has a first pulse 601 during a first time period T1 and a second pulse 602 during a second period T2 adjacent to the first time period T1. The switch 208 is adapted to close and open in response to the first pulse 601. The switch 208 is adapted to close and open in response to the second pulse 602. The controller 210 is configured to set a duration of the first time period T1 different from a duration of the second time period T2.

The pulse modulation signal 211 has a pulse width PW1-PW4 for each pulse 601-604 in the pulse modulation signal 211. The controller 210 is configured to control the pulse width PW1-PW4 to control an intensity of the pulse of light 204. For example, the controller 210 adjusts the pulse width PW1-PW4 for each pulse 601-604 to obtain the same duty cycle for each pulse. The duty cycle is the ratio between the pulse width and the time period. To have the same duty cycle for pulse 602 as for pulse 601, the controller 210 makes the pulse width PW2 shorter than the pulse width PW1, because the time period T2 is shorter than the timer period T1. To have the same duty cycle for pulse 603 as for pulse 602, the controller 210 makes the pulse width PW3 longer than the pulse width PW2, because the time period T3 is longer than the timer period T2.

For example, the multiple frequencies form a range of frequencies between the first frequency F1 and the second frequency F2. The controller 210 is configured to control the frequency content by gradually changing the pulse modulation signal 211 from the first frequency F1 to the second frequency F2.

For example, the multiple frequencies form a range of frequencies between a first frequency F1 and a second frequency F2. The controller 210 is configured to control the frequency content by selecting frequencies from the range of frequencies in a pseudo-random way, and to control start times of pulses of the pulse modulation signal 211 based on the selected frequencies.

Returning to FIG. 2, the IPL apparatus 100 optionally comprises a clock 212 connected to the controller 210. The clock 212 provides a modulated clock signal 213. The controller 210 is configured to provide the pulse modulation signal 211 based on the modulated clock signal 213.

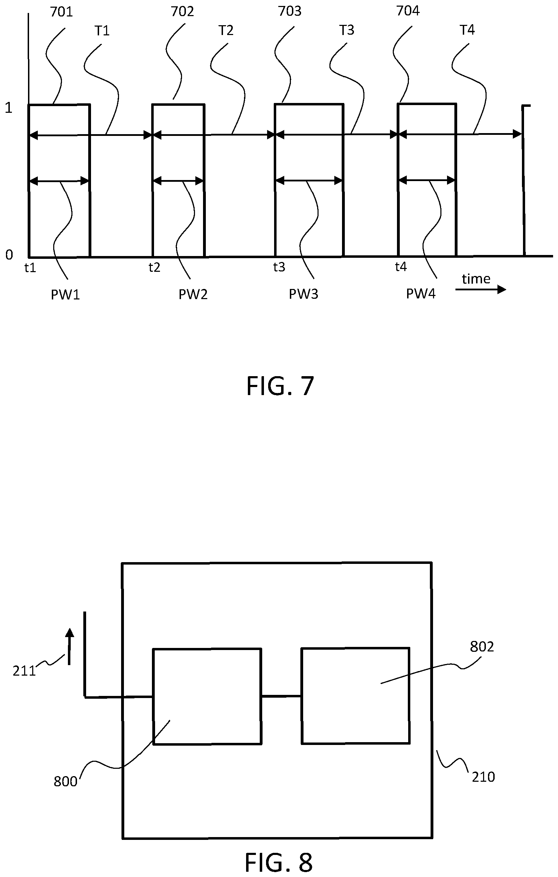

FIG. 7 depicts another example of the pulse modulation signal 211. The pulse modulation as depicted in FIG. 7 is, for example, combined with the pulse modulation as depicted in FIG. 6. FIG. 7 depicts four pulses 701-704 of the pulse modulation signal 211. Pulse 701 has time period T1 and pulse width PW1, pulse 702 has time period T2 and pulse width PW2, pulse 703 has time period T3 and pulse width PW3, and pulse 704 has time period T4 and pulse width PW4. The time periods T1-T4 all have the same duration. The pulses 701-704 have an amplitude of 1. In response to the pulses 701-704, the switch 208 closes to allow the electric current 205 to flow via the switch 208. When the pulse modulation signal 211 is 0 in between the pulses 701-704, the switch 208 is open to block the electric current 205 to flow via the switch 208.

The controller 210 is configured to control the frequency content by controlling the pulse widths PW1-PW4. PW1 has a different duration than PW2. PW2 has a different duration than PW3. As the pulse widths PW1-PW4 have different durations, whereas the time periods T1-T4 have the same duration, the pulses 701-704 have different duty cycles.

As shown in FIG. 7, the pulse modulation signal 211 has a first pulse 701 during a first time period T1 and a second pulse 702 during a second period T2 adjacent to the first time period T1. The switch 208 is adapted to close and open in response to the first pulse 701. The switch 208 is adapted to close and open in response to the second pulse 702. The controller 210 is configured to set a duration of the first pulse width PW1 different from a duration of the second pulse width PW2.

For example, the multiple frequencies form a range of frequencies between the first frequency F1 and the second frequency F2. The controller 210 is configured to control the frequency content by gradually changing the pulse modulation signal 211 from the first frequency F1 to the second frequency F2.

For example, the multiple frequencies form a range of frequencies between a first frequency F1 and a second frequency F2. The controller 210 is configured to control the frequency content by selecting frequencies from the range of frequencies in a pseudo-random way, and to control start times of pulses of the pulse modulation signal 211 based on the selected frequencies.

Returning to FIG. 2, the IPL apparatus 100 optionally comprises a clock 212 connected to the controller 210. The clock 212 provides a modulated clock signal 213. The controller 210 is configured to provide the pulse modulation signal 211 based on the modulated clock signal 213.

FIG. 8 depicts the controller 210 according to a second embodiment. The controller 210 comprises a processor 800 and a memory 802. The memory 802 is a computer-readable storage medium. The memory 802 stores a computer program product. The computer program product comprises instructions which, when executed by the controller 210 of the IPL apparatus 100, cause the controller 210 to carry out the method as depicted in FIG. 8.

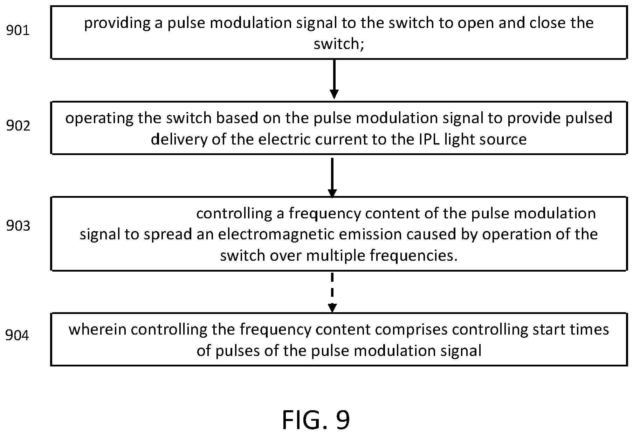

FIG. 9 depicts a method for controlling the switch 208 of the IPL apparatus 100 according to a third embodiment. The switch 208 is electrically connected to the power source 206 and the IPL light source 202 of the IPL apparatus 100. The IPL light source 202 is adapted to generate the pulse of light 204 in response to receiving the electric current 205 from the power source 206. The power source 206 is adapted to provide the electric current 205 to the IPL light source 202. The method comprises, at 901, providing a pulse modulation signal 211 to the switch 208 to open and close the switch 208. The method comprises, at 902, operating the switch 208 based on the pulse modulation signal 211 to provide pulsed delivery of the electric current 205 to the IPL light source 202. The method comprises, at 903, controlling a frequency content of the pulse modulation signal 211 to spread an electromagnetic emission caused by operation of the switch 208 over multiple frequencies. For example, the pulse modulation signal 211 comprises a spread spectrum modulation.

Optionally, the method comprises, at 904, that controlling the frequency content comprises controlling start times of pulses of the pulse modulation signal 211.

The processor 800 is adapted to perform the data processing. The processor 800 can be implemented in numerous ways, with software and/or hardware, to perform the various functions required. The processor 800 may employ one or more microprocessors that may be programmed using software (e.g., microcode) to perform the required functions. The processor 800 may be implemented as a combination of dedicated hardware to perform some functions and one or more programmed microprocessors and associated circuitry to perform other functions.

The processor 800 may include circuitry. Examples of circuitry that may be employed include, but are not limited to, conventional microprocessors, application specific integrated circuits (ASICs), and field-programmable gate arrays (FPGAs). Thus, the processor 800 may be embodied as a digital and/or analog processing system.

In various implementations, the memory may comprise one or more storage media such as volatile and non-volatile computer memory such as RAM, PROM, EPROM, and EEPROM. The storage media may be encoded with one or more programs that, when executed on processor 800 of the controller 210, perform the required functions. Various storage media may be fixed within the processor 800 or controller 210 or may be transportable, such that the one or more programs stored thereon can be loaded into the processor 800.

Functions implemented by the processor 800 may be implemented by a single processor or by multiple separate processors which may together be considered to constitute a "processor". Such a processor may in some cases be remote from each other and communicate with each other in a wired or wireless manner.

The computer program may be stored/distributed on a suitable medium, such as an optical storage medium or a solid-state medium supplied together with or as part of other hardware, but may also be distributed in other forms, such as via the Internet or other wired or wireless telecommunication systems. The processor 800 may be configured to execute the computer program, causing the IPL apparatus 100 to perform the method according to the embodiment of FIG. 8.

Variations to the disclosed embodiments can be understood and effected by those skilled in the art in practicing the principles and techniques described herein, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An intense pulsed light, IPL, apparatus (100), comprising:
an IPL light source (202) adapted to generate a pulse of light (204) in response to receiving an electric current (205);
a power source (206) adapted to provide the electric current (205) to the IPL light source (202);
a switch (208) electrically connected to the power source (206) and the IPL light source (202);
a controller (210) configured to provide a pulse modulation signal (211) to the switch (208) to open and close the switch (208),
wherein the switch (208) is adapted to provide pulsed delivery of the electric current (205) to the IPL light source (202) in response to the pulse modulation signal (211),
wherein the controller (210) is configured to control a frequency content of the pulse modulation signal (211) to spread an electromagnetic emission caused by operation of the switch (208) over multiple frequencies.

2. The IPL apparatus (100) according to claim 1, wherein the pulse modulation signal (211) comprises a spread spectrum modulation.

3. The IPL apparatus (100) according to claim 1 or 2, wherein the controller (210) is configured to control the frequency content by controlling start times (t1-t4) of pulses of the pulse modulation signal (211).

4. The IPL apparatus (100) according to any one of the preceding claims,
wherein the pulse modulation signal (211) has a first pulse (601) during a first time period (T1), and a second pulse (602) during a second period (T2) adjacent to the first time period (T1),
wherein the switch (208) is adapted to close and open in response to the first pulse (601),
wherein the switch (208) is adapted to close and open in response to the second pulse (602),
wherein the controller (210) is configured to set a duration of the first time period (T1) different from a duration of the second time period (T2).

5. The IPL apparatus (100) according to any one of the preceding claims,
wherein the pulse modulation signal (211) has a pulse width (PW1-PW4) for each pulse (601-604) in the pulse modulation signal (211),
wherein the controller (210) is configured to control the frequency content by controlling the pulse widths (PW1-PW4).

6. The IPL apparatus (100) according to any one of the preceding claims,
wherein the multiple frequencies form a range of frequencies between a first frequency (F1) and a second frequency (F2),
wherein the controller (210) is configured to control the frequency content by gradually changing the pulse modulation signal (211) from the first frequency (F1) to the second frequency (F2).

7. The IPL apparatus (100) according to any one of the preceding claims,
wherein the multiple frequencies form a range of frequencies between a first frequency (F1) and a second frequency (F2),
wherein the controller (210) is configured to:
control the frequency content by selecting frequencies from the range of frequencies in a pseudo-random way, and
control start times of pulses of the pulse modulation signal (211) based on the selected frequencies.

8. The IPL apparatus (100) according to any one of the preceding claims, comprising a clock (212) connected to the controller (210),
wherein the clock (212) provides a modulated clock signal (213),
wherein the controller (210) is configured to provide the pulse modulation signal (211) based on the modulated clock signal (213).

9. The IPL apparatus (100) according to any one of the preceding claims, wherein the frequency content is in the range of 10 kHz and higher, for example, 10 kHz to (500) kHz, for example 50 kHz to (500) kHz.

10. The IPL apparatus (100) according to any one of the preceding claims, wherein a duration of the pulse of light (204) is in the range of 2 ms to 50 ms.

11. An electric circuit (200) for use in an intense pulsed light, IPL, apparatus (100),
wherein the IPL apparatus (100) has an IPL light source (202) adapted to generate a pulse of light (204) in response to receiving an electric current (205),
wherein the electric circuit (200) comprises:
a power source (206) adapted to provide the electric current (205) to the IPL light;
a switch (208) electrically connected to the power source (206) and the IPL light source (202);
a controller (210) configured to provide a pulse modulation signal (211) to the switch (208) to open and close the switch (208),
wherein the switch (208) is adapted to provide pulsed delivery of the electric current (205) to the IPL light source (202) in response to the pulse modulation signal (211),
wherein the controller (210) is configured to control a frequency content of the pulse modulation signal (211) to spread an electromagnetic emission caused by operation of the switch (208) over multiple frequencies.

12. A method for controlling a switch (208) of an intense pulsed light, IPL apparatus (100), wherein the switch (208) is electrically connected to a power source (206) and an IPL light source (202) of the IPL apparatus (100),
wherein the IPL light source (202) is adapted to generate a pulse of light (204) in response to receiving an electric current (205) from the power source (206),
wherein the power source (206) is adapted to provide the electric current (205) to the IPL light source (202),
wherein the method comprises:
providing (901) a pulse modulation signal (211) to the switch (208) to open and close the switch (208);
operating (902) the switch (208) based on the pulse modulation signal (211) to provide pulsed delivery of the electric current (205) to the IPL light source (202);
controlling (903) a frequency content of the pulse modulation signal (211) to spread an electromagnetic emission caused by operation of the switch (208) over multiple frequencies.

13. The method according to claim 12, wherein the pulse modulation signal (211) comprises a spread spectrum modulation.

14. The method according to claim 12 or 13, wherein controlling the frequency content comprises controlling (904) start times of pulses of the pulse modulation signal (211).

15. A computer program product, comprising instructions which, when executed by a controller (210) of an IPL apparatus (100), cause the controller (210) to carry out the method of claims 12-14.
